# FASCICULE DE BREVET EUROPEEN

(11) **EP 3 332 697 B1**
(45) Date de publication et mention de la délivrance du brevet: **14.08.2019**
(21) Numéro de dépôt: 17200711.4
(22) Date de dépôt: 09.11.2017
(51) Int. Cl.: A61B 5/00, A61B 5/11

(54) **PROCÉDÉ DE DÉTECTION ET DE CALCUL DE DURÉE D'UN SAUT**
VERFAHREN ZUR ERKENNUNG UND BERECHNUNG DER DAUER EINES SPRUNGS
METHOD FOR DETECTING AND CALCULATING THE DURATION OF A JUMP

(30) Priorité: 12.12.2016 EP 16203365
(43) Date de publication de la demande: 13.06.2018
(73) Titulaire: The Swatch Group Research and Development Ltd, 2074 Marin (CH)
(72) Inventeur: Germiquet, Christophe, 2515 Prêles (CH); Ferri, Yvan, 1004 Lausanne (CH); Willemin, Michel, 2515 Prêles (CH)
(74) Mandataire: ICB SA

(56) Documents cités:
- JP-A- 2012 217 584
- TIEN JUNG LEE ET AL: "Automatic jump detection in skiing/snowboarding using head-mounted MEMS inertial and pressure sensors", PROCEEDINGS OF THE INSTITUTION OF MECHANICAL ENGINEERS, PART P: JOURNAL OF SPORTS ENGINEERING AND TECHNOLOGY,, vol. 229, no. 4, 1 janvier 2015 (2015-01-01), pages 278-287, XP008184828,
- ROBERTS-THOMSON CLAIRE L ET AL: "Jump detection using fuzzy logic", 2014 IEEE SYMPOSIUM ON COMPUTATIONAL INTELLIGENCE FOR ENGINEERING SOLUTIONS (CIES), IEEE, 9 décembre 2014 (2014-12-09), pages 125-131, XP032723870, DOI: 10.1109/CIES.2014.7011841

## Description

### Domaine de l'invention

L'invention se rapporte à un procédé de détection et de calcul de la durée d'un saut. Le procédé est adapté à des sauts mettant en oeuvre des moyens de déplacement permettant de prendre de l'élan avant le saut, tels que des skis, un snowboard, des rollers, un vélo, un skateboard, etc. Le procédé est également adapté à des sauts ne mettant pas en oeuvre ce genre de moyens de déplacement, par exemple des sauts dans l'eau depuis une falaise, un plongeoir ou un pont.

Par durée, on entend la différence entre l'instant de réception et l'instant où le sportif débute le saut. Dans le cas d'un saut dans l'eau, on appelle réception l'entrée du sportif dans l'eau.

### Arrière-plan de l'invention

On connaît du document US2002/0116147 un procédé de détection et d'analyse d'un saut au moyen d'une unité de mesure montée sur des moyens de déplacement utilisés par un sportif pour prendre de l'élan avant le saut et se trouvant en contact avec le sol avant et après le saut. Les moyens de déplacement sont par exemple des skis ou un snowboard. Une unité de calculs, par exemple une montre portée par le sportif, permet de déterminer et d'afficher des paramètres du saut, notamment la durée du saut, à partir des mesures réalisées par l'unité de mesure. Plus précisément, l'unité de mesure relève des vibrations des moyens de déplacement, pour détecter lorsque les moyens de déplacement quittent le sol et retournent au sol, ce qui permet de détecter un saut et de calculer sa durée.

Ce procédé présente l'inconvénient de nécessiter l'utilisation d'une unité de mesure à fixer aux moyens de déplacement, afin de mesurer les vibrations qu'ils subissent. De plus, le procédé ne permet pas de calculer la durée d'un saut effectué sans moyens de déplacement en contact avec le sol avant et après le saut, par exemple un saut dans l'eau depuis une falaise.

L'article de Tien Jung Lee et al 'Automatic jump detection in skiing/ snowboarding using head-mounted MEMS inertial and pressure sensors' J. Sports Engineering and Technology 2015, Vol. 229(4) 278-287 décrit un procédé de détection d'un saut basé sur des mesures d'accélération.

### Résumé de l'invention

Le but de la présente invention est de pallier tout ou partie les inconvénients cités précédemment.

A cet effet, l'invention se rapporte à un procédé de détection et de calcul de durée d'un saut effectué par un individu, selon les revendications 1 à 4.

On entend par g, l'accélération de la pesanteur à la surface de la terre, c'est-à-dire 9,80665 m.s⁻².

Par mesure d'accélération, on entend la norme d'un vecteur d'accélération à 3 composantes, c'est-à-dire la racine carrée de la somme des carrés des composantes.

L'invention tire profit du constat qu'une réception au sol ou dans l'eau consécutive à un saut est à l'origine d'un fort pic d'accélération observé au sein des données mesurées par un accéléromètre triaxial. Une détection d'un pic d'accélération est donc un indice qu'un saut a été effectué. Pour confirmer qu'un saut a bien été effectué et que le pic d'accélération ne correspond pas à une fausse détection, les mesures d'accélérations précédant le pic d'accélération sont analysées. En effet, lors d'un saut, l'individu est en chute libre, donc la norme de son accélération est en théorie nulle. Une succession de mesures d'accélération proches de zéro pendant une durée suffisamment longue, suivie par un pic d'accélération suffisamment important, indique donc qu'un saut a été effectué.

En outre, le procédé comprend les étapes suivantes :
- une détection d'un instant associé au début du saut, correspondant à la première mesure de la succession de mesures d'accélération comprises entre 0 et 0,5g,
- un calcul d'une durée du saut par différence de l'instant associé à la réception et de l'instant associé au début du saut.

Lorsqu'une phase de saut a été détectée, les mesures d'accélération sont utilisées pour calculer la durée du saut. La première mesure des mesures successives d'accélération comprises entre 0 et 0,5g correspond au moment où l'individu a débuté le saut. Quant au pic d'accélération, il correspond au moment où l'individu s'est réceptionné. En soustrayant la première mesure et l'instant du pic d'accélération, la durée du saut peut donc être calculée.

De plus, l'étape de détection d'un instant associé à une réception comprend une sous-étape de détection d'un pic de pression d'amplitude supérieure à une deuxième amplitude seuil et de durée inférieure à une deuxième durée seuil, à partir de mesures de pression fournies par un capteur de pression embarqué dans la montre, et une sous-étape de comparaison de l'instant associé au pic d'accélération et de l'instant associé au pic de pression.

Les caractéristiques précitées tirent profit du constat qu'une réception consécutive à un saut est à l'origine d'un fort pic de pression observé au sein des données mesurées par un capteur de pression. Une détection d'un pic de pression est donc un indice d'une réception qui permet de confirmer qu'un saut a bien eu lieu par corrélation avec le pic d'accélération.

Outre les caractéristiques précitées, le procédé selon l'invention peut comprendre les caractéristiques suivantes, prises seules ou en combinaison selon toutes les combinaisons techniquement possibles.

Dans un mode de réalisation non limitatif, la deuxième amplitude seuil est supérieure à 2 hectopascals et la deuxième durée seuil est supérieure à 0.1 seconde.

Dans un mode de réalisation non limitatif, la première durée seuil est supérieure à 0.5 seconde.

Dans un mode de réalisation non limitatif, la première amplitude seuil est supérieure à 2g.

### Description sommaire des dessins

D'autres particularités et avantages ressortiront clairement de la description qui en est faite ci-après, à titre indicatif et nullement limitatif, en référence aux dessins annexés, dans lesquels :
- La figure 1 représente une montre électronique permettant la mise en oeuvre du procédé selon un mode de réalisation de l'invention.
- La figure 2 illustre un exemple de trajectoire d'un saut en ski.
- La figure 3 montre une courbe représentant des mesures d'accélération, superposée à une courbe représentant des mesures de pression, les mesures étant réalisées lors du saut de la figure 2.
- La figure 4 montre un diagramme fonctionnel représentatif des étapes du procédé.

### Description détaillée des modes de réalisation préférés

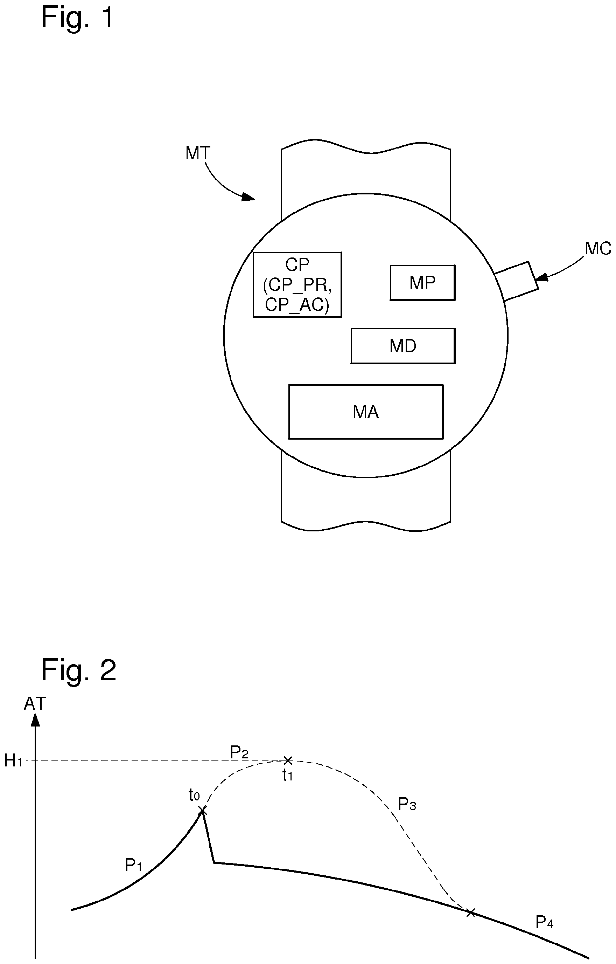

Le procédé METH selon l'invention est intégralement mis en oeuvre par une montre MT électronique portée par un individu réalisant un saut. Dans un mode de réalisation non limitatif montré à la figure 1, la montre MT comporte :
- un ensemble de capteurs CP, dont un accéléromètre CP_AC et un capteur de pression CP_PR (ou altimètre)
- une mémoire MD pour enregistrer des mesures réalisées par les capteurs CP. Les mesures sont avantageusement enregistrées dans la mémoire de manière glissante, selon le principe FIFO (« first in, first out »)
- un microprocesseur MP pour traiter les informations contenues dans la mémoire MD
- des moyens d'affichage MA numériques ou analogiques, pour afficher les résultats des calculs réalisés par le microprocesseur MP
- des moyens d'activation MC, mécaniques, électroniques ou tactiles, des capteurs CP, de la mémoire MD, du microprocesseur MP et des moyens d'affichage MA, permettant de déclencher le procédé PR.

La figure 2 montre un exemple de trajectoire d'un saut, en l'occurrence un saut en ski. Dans une première phase P₁, l'individu est en contact avec le sol. A un temps t₀, l'individu décolle du sol, et dans une deuxième phase P₂, l'individu est en phase de montée. A un temps t₁, l'individu atteint une altitude H₁ maximale, et dans une troisième phase P₃, l'individu est en phase de descente. A un temps t₂, l'individu se réceptionne au sol, et dans une quatrième phase P₄, l'individu est à nouveau en contact avec le sol.

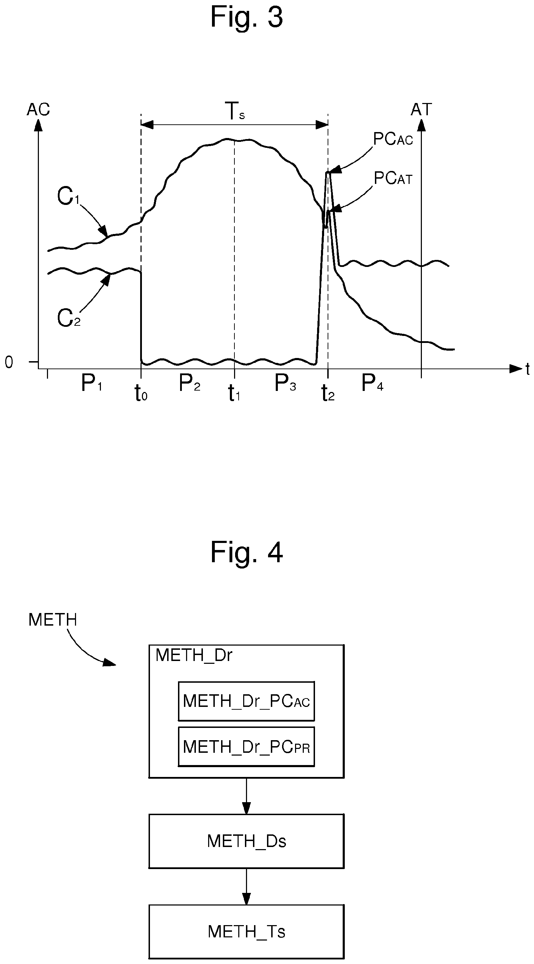

La figure 3 montre une courbe C₁ représentant des mesures d'altitude AT calculées à partir des mesures de pression relevées par le capteur de pression CP_PR de la montre MT, en fonction du temps t, et en particulier lors des quatre phases P₁, P₂, P₃ et P₄. Une courbe C₂ représentant l'accélération AC mesurée par l'accéléromètre CP_AC de la montre MT en fonction du temps t, est superposée à la courbe d'altitude C₁.

Une première étape du procédé METH selon l'invention consiste à détecter qu'un saut a été effectué (étape METH_Dr sur la figure 4). Plus particulièrement, l'étape de détection METH_Dr comporte une première sous-étape METH_Dr_PC_{AC} consistant à détecter un pic d'accélération PC_{AC} dans les mesures fournies par l'accéléromètre CP_AC. En effet, lorsqu'un saut est effectué, on observe un pic d'accélération PC_{AC} à l'instant t₂, c'est-à-dire à la réception au sol de l'individu. Lorsqu'un tel pic PC_{AC} est détecté, il est comparé à une valeur seuil au-delà de laquelle il est décidé que le pic PC_{AC} correspond bien à une réception au sol consécutive à un saut.

Dans un mode de réalisation, pour confirmer que ce pic d'accélération PC_{AC} correspond bien à une réception consécutive à un saut, l'étape de détection METH_Dr comporte également une deuxième sous-étape METH_Dr_PC_{PR} consistant à détecter un pic de pression PC_{PR} dans les mesures fournies par le capteur de pression CP_PR. Le pic d'altitude PC_{AT} correspondant est représenté à la figure 3. Si un saut a bien eu lieu, un tel pic de pression PC_{PR} devrait être relevé à un instant sensiblement identique à celui auquel le pic d'accélération PC_{AC} a été détecté. Les instants correspondants au pic de pression PCPR et au pic d'accélération PC_{AC} sont donc comparés. Si la norme de la différence entre ces instants est inférieure à une valeur seuil, par exemple 0.5 seconde, alors il est décidé que les pics PC_{PR}, PC_{AC} correspondent bien à une réception au sol consécutive à un saut.

Une deuxième étape du procédé METH selon l'invention consiste à détecter une phase de saut (étape METH_Ds), par détection, dans une fenêtre temporelle s'achevant à l'instant t₂ déterminé précédemment, d'une succession de mesures d'accélération AC comprises entre 0 et 0,5g pendant une durée supérieure à une première durée seuil t_{S}. Pour cela, les mesures d'accélération AC dans une fenêtre temporelle précédant le pic d'accélération P_{AC}, par exemple une fenêtre temporelle d'une dizaine de secondes, sont analysées. Plus précisément, l'étape METH_Ds de détection d'une phase de saut comporte un calcul des normes des mesures d'accélération de la fenêtre temporelle. Il est alors possible de déterminer si le pic d'accélération PC_{AC} est précédé d'une succession de mesures comprises entre 0 et 0,5g pendant une durée supérieure à la première durée seuil t_{S}. Si cela est le cas, il est confirmé qu'un saut a bien eu lieu et que le pic d'accélération PC_{AC} correspond à une réception au sol. En effet, lors de la troisième phase P₃, l'individu est en chute libre et subit donc une accélération d'environ 0g. La première de la succession de mesures comprises entre 0 et 0,5g correspond à un temps t₀ attribué au début du saut.

Dans une troisième étape du procédé, la durée Ts du saut est calculée (étape METH_Ts). Pour cela, l'instant t₂ associé à la réception est retranché à l'instant t₀ associé au début du saut.

On remarque donc que l'étape METH_Ds de détection d'une phase de saut contribue non seulement à confirmer qu'un saut a bien eu lieu, mais également à calculer la durée de ce saut.

Bien entendu, la présente invention ne se limite pas à l'exemple illustré mais est susceptible de diverses variantes et modifications qui apparaîtront à l'homme de l'art.

## Revendications

1. Procédé (METH) de détection et de calcul de durée d'un saut effectué par un individu, mis en oeuvre par une montre électronique comportant les étapes suivantes :
- une détection (METH_Dt₂) d'un instant (t₂) associé à une réception consécutive au saut, cette étape comprenant une sous-étape de détection (METH_Dt₂_PC_{AC}) d'un pic d'accélération (PC_{AC}) d'amplitude supérieure à une première amplitude seuil, au sein de mesures d'accélération (AC) fournies par un accéléromètre (CP_AC) trois axes embarqué dans une montre (MT) portée au poignet de l'individu,
- une détection (METH_Ds) d'une phase de saut, par détection, dans une fenêtre temporelle s'achevant à l'instant (t₂) de réception, d'une succession de mesures d'accélération (AC) entre 0 et 0,5g pendant une durée supérieure à une première durée seuil (t_{S}), g étant l'accélération de la pesanteur à la surface de la terre,
- une détection (METH_Dt₁) d'un instant (t₀) associé au début du saut, correspondant à la première mesure de la succession de mesures d'accélération (AC) comprises entre 0 et 0,5g,
- un calcul (METH_Ts) d'une durée (Ts) du saut par différence de l'instant (t₂) associé à la réception et de l'instant (t₀) associé au début du saut,
l'étape de détection (METH_Dt₂) d'un instant (t₂) associé à une réception comprenant une sous-étape de détection (METH_Dt₂_PC_{PR}) d'un pic de pression (PC_{PR}) d'amplitude supérieure à une deuxième amplitude seuil et de durée inférieure à une deuxième durée seuil, à partir de mesures de pression (PR) fournies par un capteur de pression (CP_PR) embarqué dans la montre (MT), et une sous-étape de comparaison de l'instant associé au pic d'accélération (PC_{AC}) et de l'instant associé au pic de pression (PC_{PR})

2. Procédé (METH) selon la revendication précédente, dans lequel la deuxième amplitude seuil est supérieure à 2 hectopascals et la deuxième durée seuil est inférieure à 0.1 seconde.

3. Procédé (METH) selon l'une des revendications précédentes, dans lequel la première durée seuil est supérieure à 0.5 seconde.

4. Procédé (METH) selon l'une des revendications précédentes, dans lequel la première amplitude seuil est supérieure à 2g.

## Patentansprüche

1. Verfahren (METH) zum Erkennen und Berechnen der Dauer eines von einer Person ausgeführten Sprungs, das in einer elektronischen Uhr ausgeführt wird und das die folgenden Schritte umfasst:
- Erkennen (METH_Dt₂) eines Zeitpunkts (t₂), der einer auf den Sprung folgenden Landung zugeordnet ist, wobei dieser Schritt einen Unterschritt zum Erkennen (METH_Dt₂_PC_{AC}) einer Beschleunigungsspitze (PC_{AC}) mit einer Amplitude höher als eine erste Schwellenamplitude innerhalb von Beschleunigungsmessungen (AC), die von einem dreiachsigen Beschleunigungsmesser (CP_AC) bereitgestellt werden, der in eine am Handgelenk der Person getragene Uhr (MT) eingebaut ist, umfasst,
- Erkennen (METH_Ds) einer Sprungphase durch Erkennen einer Folge von Beschleunigungsmessungen (AC) zwischen 0 und 0,5 g in einem zu dem Landungszeitpunkt (t₂) endenden Zeitfenster während einer Dauer, die größer als eine erste Schwellendauer (t_{S}) ist, wobei g die Erdbeschleunigung an der Erdoberfläche ist,
- Erkennen (METH_Dt₁) eines Zeitpunkts (t₀), der dem Beginn des Sprungs zugeordnet ist und der ersten Messung der Folge von Beschleunigungsmessungen (AC) im Bereich von 0 bis 0,5 g entspricht,
- Berechnen (METH_Ts) einer Dauer (Ts) des Sprungs anhand der Differenz zwischen dem der Landung zugeordneten Zeitpunkt (t₂) und dem dem Beginn des Sprungs zugeordneten Zeitpunkt (t₀),
wobei der Schritt (METH_Dt₂) zum Erkennen eines einer Landung zugeordneten Zeitpunkts (t₂) einen Unterschritt zum Erkennen (METH_Dt₂_PC_{PR}) einer Druckspitze (PC_{PR}) mit einer Amplitude größer als eine zweite Schwellenamplitude und von einer Dauer geringer als eine zweite Schwellendauer anhand von Druckmessungen (PR), die von einem in die Uhr (MT) eingebauten Drucksensor (CP_PR) bereitgestellt werden und einen Unterschritt zum Vergleichen des Zeitpunkts, der der Beschleunigungsspitze (PC_{AC}) zugeordnet ist, und des Zeitpunkts, der der Druckspitze (PC_{PR}) zugeordnet ist, umfasst.

2. Verfahren (METH) nach dem vorhergehenden Anspruch, wobei die zweite Schwellenamplitude größer als 2 Hektopascal ist und die zweite Schwellendauer kleiner als 0,1 Sekunden ist.

3. Verfahren (METH) nach einem der vorhergehenden Ansprüche, wobei die erste Schwellendauer größer als 0,5 Sekunden ist.

4. Verfahren (METH) nach einem der vorhergehenden Ansprüche, wobei die erste Schwellenamplitude größer als 2 g ist.

## Claims

1. Method (METH) for detecting and calculating the duration of a jump effected by an individual, implemented by an electronic watch comprising the following steps:
- detection (METH_Dt₂) of a moment (t₂) associated with a landing following the jump, this step comprising a sub-step of detecting (METH_Dt₂_PC_{AC}) an acceleration peak (PC_{AC}) of an amplitude greater than a first threshold amplitude, within the acceleration measurements (AC) provided by a three-axis accelerometer (CP_AC), on board a watch (MT) worn on the wrist of the individual,
- detection (METH_Ds) of a jump phase, by detection, in a temporal window finishing at the moment (t_{S}) of landing, of a succession of acceleration measurements (AC) between 0g and 0.5g during a duration greater than a first threshold duration (t_{S}), g being the acceleration due to gravity at the Earth's surface,
- detection (METH_Dt₁) of a moment (t₀) associated with the start of the jump corresponding to the first measurement of the succession of acceleration measurements (AC) between 0 and 0.5g,
- calculation (METH_Ts) of a duration (Ts) of the jump by the difference of the moment (t₂) associated with the landing and the moment (t₀) associated with the start of the jump,
the step of detecting (METH_Dt₂) a moment (t₂) associated with a landing comprising a sub-step of detecting (METH_Dt₂_PC_{PR}) a pressure peak (PC_{PR}) of an amplitude greater than a second threshold amplitude and of lesser duration than a second threshold duration, from pressure measurements (PR) provided by a pressure sensor (CP_PR) on board the watch (MT), and a sub-step of comparing the moment associated with the acceleration peak (PC_{AC}) and the moment associated with the pressure peak (PC_{PR}).

2. Method (METH) according to the preceding claim, in which the second threshold amplitude is greater than 2 millibars and the second threshold duration is less than 0.1 second.

3. Method (METH) according to one of the preceding claims, in which the first threshold duration is greater than 0.5 second.

4. Method (METH) according to one of the preceding claims, in which the first threshold amplitude is greater than 2g.
